# EUROPEAN PATENT SPECIFICATION

(11) **EP 0 471 794 B1**
(45) Date of publication and mention of the grant of the patent: **02.10.1996**
(21) Application number: 90909103.5
(22) Date of filing: 11.05.1990
(51) Int. Cl.: A61K 31/35, A61K 31/54

(54) **ANTIVIRAL THERAPY USING THIAZINE AND XANTHENE DYES**
ANTIVIRALE THERAPIE UNTER VERWENDUNG VON THIAZIN- UND XANTHENFARBSTOFFEN
THERAPIE ANTIVIRALE UTILISANT DES COLORANTS A BASE DE THIAZINE ET DE XANTHENE

(30) Priority: 11.05.1989 US 350383; 03.08.1989 US 389007
(43) Date of publication of application: 26.02.1992
(73) Proprietor: Oklahoma Medical Research Foundation, Oklahoma City Oklahoma 73104 (US); SCHINAZI, Raymond F., Decatur, GA 30033 (US)
(72) Inventor: SCHINAZI, Raymond, F., Decatur, GA 30033 (US); FLOYD, Robert, A., Oklahoma City, OK 73120 (US)
(74) Representative: Hallybone, Huw George
(86) International application number: US9002659
(87) International publication number: WO9013296

(56) References cited:
- DE-A- 3 341 001
- FR-A- 2 118 030
- US-A- 4 305 390
- ARCH OPHTHALMOL, vol. 105, no. 10, October 1987, pages 1415-1417; M.I. ROAT et al.: "The antiviral effects of rose bengal and fluorescein"
- CHEMICAL ABSTRACTS, vol. 87, no. 20, 14th November 1977, page 304, abstract no. 157053p, Columbus, Ohio, US; ANON: "Methylene blue; oral use in dogs and cats"
- DERMATOL. MONATSSCHR., Vol. 163, No. 7, 1977; H. MEFFERT et al.: "Photodynamic treatment of herpes simplex", pages 563-564 see Abstract.
- ARCH. OPHTHAMOL., Vol. 105, No.10, 1987; M.I. ROAT et al.: "The antiviral effects of rose bengal and fluoroscein", pages 1415-1417 see Abstract
- JPN. J. OPHTHALMOL. (Japan), Vol. 21, No. 3, 1977; Y. TANO et al.: "Photodynamic inactivation of herpes simplex virus", pages 392-399 see Abstract
- J. CLIN. INVEST. Vol. 65, February 1980; L.E. SCHNIPPER et al.: "Mechanisms of photodynamic in-activation of herpes simplex viruses", pages 432-438
- J. CLIN. MICROBOL., Vol. 17, No. 2, February 1983; J.A. BAPYLAK et al.: "Photodynamic inactivation of pseudorabies virus with methylene blue dye, light AND ELECTRICITY", pages 374-376

## Description

### Background of the Invention

The United States government has rights in this invention by virtue of National Institutes of Health grant No. CA42854.

This invention is generally in the area of methods for the treatment of viral diseases, and more specifically relates to the treatment of AIDS using either thiazine or xanthene dyes.

Methylene blue, 3,7-Bis(dimethylamino)-phenothiazin-5-ium chloride, C₁₆H₁₈ClN₃S, is a dark green or blue thiazine dye which was first isolated in 1876. It is FDA approved for oral administration and has been reported to be effective as an antiseptic, disinfectant, and antidote for cyanide and nitrate poisoning. For over 50 years it has been known that methylene blue is reduced by mitochondria to leukodye which is then auto-oxidized back to methylene blue by oxygen, yielding H₂O₂. This is the probable mechanism by which methylene blue, injected i.v. at a dose of 1 mg/kg body weight, is effective in the treatment of methemoglobinemia, a clinical disorder where more than 1% of the hemoglobin in the blood has been oxidized to Fe³⁺. Kelner and Alexander reported in J. Biol. Chem. 260(28), 15168-15171 (1985), that methylene blue oxidizes glutathione directly when it is reduced by NADPH, rather than via the H₂O₂.

Methylene blue, in the presence of light, has been reported to damage DNA, probably by destroying or cleaving the DNA at the guanine residues. Simon and Van Vunakis, Arch. Biochem. Biophys. 105, 197-206 (1964), noted that the effect of several photoactive dyes and light is dependent on the concentration of the dye, as well as light wavelength and intensity, and can be correlated with uptake of oxygen and decrease in ultraviolet absorbance by guanine derivatives. Kornhauser, et al., Photochem. Photobiol. 18, 63-69 (1973) attempted to characterize the changes in guanosine following exposure to methylene blue and light using thin layer chromatographic analytical techniques.

Others have also attempted, without success, to analyze the actual mechanism of the effect of methylene blue and light on DNA. Friedmann and Brown, Nucleic Acids Res. 5, 615-622 (1978), showed that methylene blue and light caused lesions at deoxyguanosines in DNA and that subsequent exposure to piperidine caused strand rupture. They hypothesized that cyclo-addition occurred at various positions in the purine ring, rendering the DNA susceptible to base catalyzed cleavage following modification of the other nucleoside bases.

Waskell, et al., reported in Biochim. Biophys. Acta 129, 49-53 (1966), that extensive irradiation of polynucleotides in the presence of methylene blue causes extensive destruction of guanosine, leaving ribose, guanidine, ribosylurea, and free urea. They postulated that the destruction of the guanosine residues was the mechanism for a previous observation by Sastry, et al., Biochim. Biophys. Acta 129, 42 (1966), that, in vitro, methylene blue and irradiation inactivates Tobacco Mosaic Virus (TMV) RNA, rendering the virus uninfective. Singer and Fraenkel-Conrat, have also reported, in Biochem. 4, 2446-2450 (1966), that another type of dye, thiopyronin (where the ring N is replaced by CH), and proflavin, cause inactivation of TMV RNA in the presence of light. This is also the probable mechanism for the observation that topical administration of a 0.1% solution of methylene blue in conjunction with polychromatic light photoinactivates viruses such as herpes simplex, as referenced in American Hospital Formulary Service 92:00 Unclassified Therapeutic Agents, page 2176 editor, Gerald K. McEvoy (American Society of Hospital Pharmacists, Inc. 1981 revised 1988). Other observations have been made showing in vitro inactivation of viruses using light, methylene blue, and electricity, as reported in J. Clin.Microbiol., 17(2), 374-376 (1983), by Badylak, et al., (pseudorabies virus) and Proc.Soc.Exper.Biol.Med. 161,204-209 (1979) by Swartz, et al., (Herpes simplex).

The xanthene dyes are derivatives of the compound xanthene which has the following chemical structure: 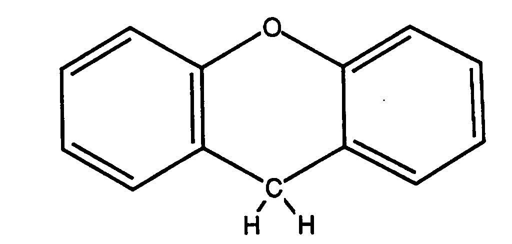 The xanthene dyes fall into three major categories: the fluorenes or amino xanthenes, the rhodols or aminohydroxyxanthenes, and the fluorones or hydroxyxanthenes. Lillie, H. J. Conn's Biological Stains 326 (Williams & Wilkins, 9th ed. 1977). Two dyes, rose bengal and eosin Y, are members of the fluorone category which have a third aryl group attached to the central carbon atom of the xanthene structure.

Rose bengal, 4,5,6,7-Tetrachloro-3',6'-dihydroxy-2',4',5',7'-tetraiodospiro[isobenzofuran-1(3H), 9'-[9H]xanthen]-3-one dipotassium or disodium salt, C₂₀H₂Cl₄I₄K₂O₅, has the following chemical structure: 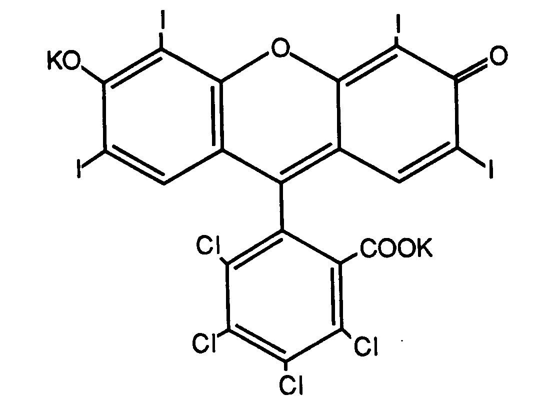 This dye has been used as a marker for Herpes keratitis. It has also been used as a bacterial stain in soil suspensions, for the negative staining of bacteria, and for spirochaetes in blood. Id. at 351. Also, it has been used in Delprat and Stowe's test (1931) for liver function, in combination with Iodine 131 for photoscanning the liver, and as a useful fluorochrome in the study of fats under ultraviolet illumination. Id. Smith and Dawson (1944) used the dye as a bacteriostatic agent in media to permit growth of soil fungi while repressing the bacteria. Id.

Eosin Y, 2',4',5',7'-tetrabromofluorescein, disodium salt, C₂₀H₆O₅Br₄Na₂, has the following structure: 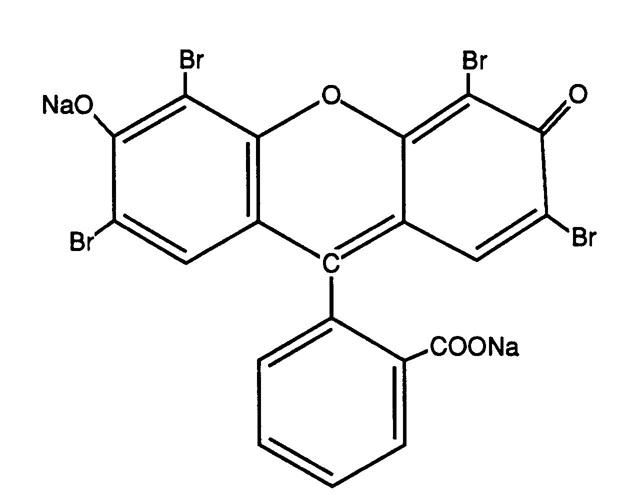 This dye is used for staining the oxyphil granules of cells, the granules having special affinity for acid dyes. Id. at 343. Also, it has been employed as a counterstain for hematoxylin and the green or blue basic dyes. Id. Eosin Y, in combination with methylene blue, has been used as a blood stain in the technique of Romanovsky and in Mann's stain to stain nerve cells, Negri bodies, anterior hypophysis, collagen, and erythrocytes. Id.

Acquired Immunodeficiency Syndrome (AIDS) is generally accepted at this time to be a consequence of infection with the retrovirus variously termed human T-lymphotropic virus type III (HTLV-III), lymphadenopathy-associated virus (LAV), AIDS associated retrovirus (ARV), or human immunodeficiency virus (HIV-1). There is considerable difficulty in diagnosing the risk of development of AIDS. AIDS is known to develop in at least 50% of the individuals infected with human immunodeficiency virus (HIV), although this percentage is suspected to be much higher.

A patient is generally diagnosed as having AIDS when a previously healthy adult with an intact immune system acquires impaired T-cell immunity. The impaired immunity usually appears over a period of eighteen months to three years. As a result of this impaired immunity, the patient becomes susceptible to opportunistic infections, various types of cancer such as Kaposi's sarcoma, and other disorders associated with reduced functioning of the immune system.

No treatment capable of preventing or curing HIV infection is currently available, although several compounds have demonstrated antiviral activity against the virus in vitro, including HPA-23, interferons, ribavirin, phosphonoformate, ansamycin, suramin, imuthiol, penicillamine rifabutin, AL-721, 3'-azido-3'-deoxythymidine (AZT), and other 2',3'-dideoxynucleosides. AZT is the only drug which has been demonstrated to prolong life in patients infected with HIV. However, AZT is quite toxic when used for periods of several months and must be discontinued even in those patients initially tolerant to the drug due to the drug causing severe anemia. See Yarchoan et al., Lancet, 575-580 (1986). Further, AZT-resistant strains of HIV have now been reported in patients undergoing treatment with AZT, Larder, B.A., Darby, G., Richman, D.D., Science 243, 1731-1734 (1989).

Many inhibitors of cellular processes, such as AZT, limit viral replication, but are at the same time quite toxic for the host as well. Most of the antiviral drugs that have been discovered so far cannot be prescribed for a prolonged period of time because of their toxicity. Accordingly, it is clear that there is a strong need for new antiviral agents, especially those with low toxicity to normal cells. More particularly, because of the high mortality of AIDS and the lack of an effective treatment for this disease, there remains a great need for development of new low toxicity agents for prophylactic use as well as long term therapy of AIDS patients.

Reference is made to the following disclosures.

Arch Ophthalmol, Volume 105, No. 10, October 1987, pages 1415 - 1417, M.I. Roat *et al*. discloses the effects of rose bengal and fluorescein sodium in the mouse herpetic keratitis model. In this model, mice are infected with HSV-1 on the eye following corneal scarification. Thus, the infection is a topical infection of the cornea.

FR-A-2118030 discloses the use of xanthene derivatives in treating coronavirus, myxovirus, arbovirus, poxvirus and herpes simplex virus infections, in non-human hosts. The experiments conducted consist of infecting mice with a virus, administering the compound under study and determining the length of time by which the survival of the mouse is increased.

DE-A-3341001 discloses a nanoparticle composition including at least 3% of a drug. In one example, methylene blue is used as a marker dye in a composition comprising an antifungal drug.

Chemical Abstracts, Volume 87, No. 20, 14th November 1977, page 304, abstract No. 157053P discloses the oral use of methylene blue in dogs and cats. However, the therapeutic utility of these drugs is called into question by this document, which states that methylene blue may cause the production of Heinz bodies, indicative of haemolytic anaemia in cats and dogs. Moreover, the effectiveness of the drug as an urinary antiseptic is called into question.

US-A-4305390 discloses the use of methylene blue, light, oxygen and electricity to inactivate herpes virus. The method of this disclosure requires that an electric field be applied, making the method difficult to use *in vivo.* Moreover, as shown in figure 9, the disclosed method is toxic to cells.

Dermatol. Monatsschr., Volume 163, No. 7, 1977, pages 563-564 discloses the inactivation of herpes simplex virus by the combination of visible light and topically applied methylene blue.

JPN. J. Ophthalmol., Volume 21, No. 3, 1977, pages 392-399 discloses the photodynamic inactivation of herpes simplex virus using topical application of antiviral drugs or dyes including toluidine blue, eosin Y, methylene blue and fluorescein sodium.

J. Clin. Invest., Volume 65, February 1980, pages 432-438 discloses the inactivation of HSV using methylene blue, light and electricity. This paper discloses only inactivation of HSV *in vitro.*

J. Clin. Microbiol., Volume 17, No. 2, February 1983, pages 374-376 discloses the inactivation of pseudorabies virus with methylene blue, light and electricity. Again, only *in vitro* data are present.

It is therefore an object of the present invention to provide methods and compositions for treatment or prevention of viral infections.

It is a further object of the present invention to provide methods and compositions for selectively inactivating HIV, both in vitro and in vivo.

### Summary of the Invention

According to the present invention there is provided a use of a xanthene or thiazine dye and a method as defined in the claims.

Compositions containing as the active agent either a thiazine dye, especially methylene blue, or xanthene dye, especially fluorones or hydroxyxanthenes, are used for the selective inactivation or inhibition of intracellular replication of specific viruses, especially human immunodeficiency virus. In the preferred embodiment of the thiazine dyes, methylene blue is the active anti-viral agent. Other useful thizaine dyes include azure A, azure C, toluidine, and thionine. In the most preferred embodiment of the xanthene dyes, the xanthene dyes are rose bengal and eosin Y, delivered orally. Selective delivery can be achieved using systems such as liposomes for delivery to macrophages and other phagocytic cells or using biodegradable controlled release implants. Examples demonstrate inactivation of human immunodeficiency virus by methylene blue, rose bengal and eosin Y. These compounds were also shown to be non-toxic in vitro to normal PBM cells and Vero cells.

### Brief Description of the Drawings

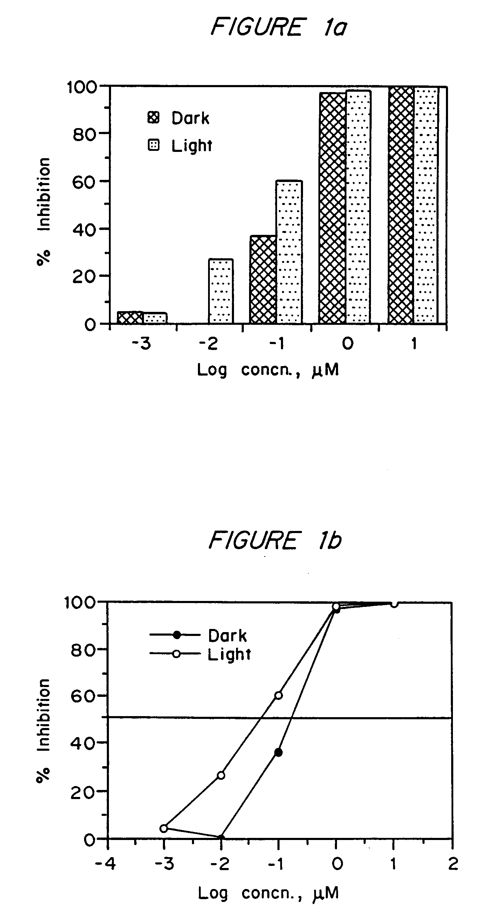

Figure 1A and 1B are graphs of the effect of methylene blue in the light and in the dark on the replication of HIV-1 in human PBM cells, % inhibition versus log concentration of methylene blue, M, Figure 1A is a bar graph; Figure 1B is a line graph.

### Detailed Description of the Invention

It has been demonstrated that the thiazine dye methylene blue plus light hydroxylates both guanosine and deoxyguanosine to yield 8-OH-guanosine (8-OH-G) and 8-OH-deoxyguanosine (8-OH-dG), respectively. The xanthene dyes similarly hydroxylate guanosine and deoxyguanosine, and non-ionizing radiation enhances the anti-viral activity of the dye. The number of guanosines in a nucleic acid strand converted to 8-OH-deoxyguanosine (8-OH-dG) or 8-OH-guanosine (8-OH-G) can be controlled through manipulation of the concentration of the dye, pH, and buffer strength. This treatment can be used to selectively, and in a controlled manner, modify the guanine bases in viral DNA and RNA, both in vitro and intracellularly. 8-OH-G is used herein to refer to both 8-OH-G and 8-OH-dG unless otherwise stated.

The production of 8-OH-G results in mutations in nucleic acid since it does not base-pair as well as the unaltered guanosine and because the bases adjacent to the 8-OH-G can be misread during replication, transcription, and translation. Viruses replicate within the host's cells, using enzymes encoded by their own genetic material and the host cell's "machinery". Replication usually occurs at a high rate, with much of the transcription and translation being involved in production of new virus. It has now been discovered that the dyes interfere with transcription and translation in the dark or in low light levels, as well as in light. Further, it has been discovered that some types of viruses are particularly sensitive to the dyes. Examples of these viruses are human immunodeficiency virus (HIV), and, to a lesser degree, Herpes simplex virus (HSV). Accordingly, the dyes can be used to treat infections by these viruses *in vivo* by interfering with the transcription and translation involved in replication of the virus in the host cells, at least in part by the mechanism whereby the dye hydroxylates the guanosine or deoxyguanosine to yield 8-OH-guanosine (8-OH-G) or 8-OH-deoxyguanosine (8-OH-dG), respectively. There is minimal effect on the host cell, presumably because of the lower rate of replication. The dyes can also be provided in combination with other known antibiotics, anti-inflammatories, antifungals, and antivirals.

Thiazine and xanthene dyes have been used in a variety of applications, as discussed in the Background of the Invention. However, the method described herein is based on the selective and controlled use of the compound, not the complete destruction of all genetic material, which would be completely inapplicable to any method wherein the goal is to preserve and minimize toxic effects to the host cells. Further, the method described herein does not require administration of exogenous light, although the results maybe enhanced by exposure to light in addition to that normally transmitted through the skin.

Examples of useful xanthene dyes are rose bengal and eosin Y. These dyes are commercially available from a number of different sources. Rose bengal can be obtained from Sigma Chemical Co., St. Louis, Mo., while eosin Y is available from Kodak Laboratory and Research Products, Rochester, N.Y.

The dyes can be applied topically or systemically for in vivo applications. Both methods of administration are approved by the Federal Drug and Food Administration for methylene blue, although methylene blue is not FDA approved at this time for any topical or in vivo application for the treatment of viral infections. Methylene blue is a thiazine dye occurring as dark blue-green crystals which is soluble in water and sparingly soluble in alcohol, forming deep blue solutions. Methylene blue injectable has a pH of 3-4.5. The pKₐ is between 0 and -1.

Drug Facts and Comparisons, page 1655 (J.B. Lippincott Co., St. Louis, MO 1989) reports that methylene blue is useful as a mild genitourinary antiseptic for cystitis and urethritis, in the treatment of idiopathic and drug-induced methemoglobemia and as an antidote for cyanide poisoning. Recommended dosages are 55 to 130 mg three times daily, administered orally. Oral absorption is 53% to 97%, averaging 74%, DiSanto and Wagner, J.Pharm.Sci.61(7), 1086-1090 (1972). Pharmacopeia states that the recommended dose is 50 to 300 mg by mouth; 1 to 4 mg/kg body weight i.v. Side effects include blue urine, occasional nausea, anemia and fever. American Hospital Formulary Service "Drug Information 88" states that the recommended i.v. dosage for children is 1 to 2 mg/kg body weight, injected slowly over several minutes, which can be repeated after an hour. 55 mg tablets are available from Kenneth Manne. 65 mg tablets are available from Star Pharmaceuticals. Methylene Blue Injection (10 mg/ml) is available from American Reagent, Harvey, Kissimmee, Pasadena.

Narsapur and Naylor reported in J.Affective Disorders 5, 155-161 (1983) that administration of methylene blue orally, at a dosage of 100 mg b.i.d. or t.i.d., or intravenously, 100 mg infused over 10 min, may be effective in treating some types of mental disorders in humans, indicating that the dye may cross the blood-brain barrier and therefore have particular applicability in the treatment of viral infections of the brain and central nervous system. Methylene blue was administered for periods of one week to 19 months to adult humans, with minimal side effects.

The American Hospital Formulary Service "Drug Information 88" reports that methylene blue is absorbed well from the GI tract, with about 75% excreted in urine and via the bile, mostly as stabilized colorless leukomethylene blue. As reported by G.E. Burrows in J.Vet.Pharmacol.Therap. 7, 225-231 (1984), the overall elimination rate constant of methylene blue, in sheep, is 0.0076 ± 0.0016 min⁻¹, with minimal methemoglobin production at doses as high as 50 mg/kg and no hematologic changes seen up to four weeks after a total dose of 30 mg/kg methylene blue. The 24 h LD₅₀ for intravenous methylene blue administered as a 3% solution was 42.3 mg/kg with 95% confidence interval limits of 37.3 to 47.9 mg/kg, demonstrating that methylene blue can be safely administered at a dosage of up to at least 15 mg/kg. As reported by Ziv and Heavner in J.Vet. Pharmacol.Therap. 7,55-59 (1984), methylene blue crosses the blood-milk barrier easily.

The method described herein for the inhibition of HIV infections in vivo requires dosages in the range producing a blood concentration of approximately 20 to 200 micromolar, or 7.5 to 75 mg/l. The usual blood volume for babies is approximately 2.5 l, for adult humans it is approximately 10 l. Taking into account the 74% oral absorption and 75% excretion of that absorbed over a period of time, and assuming the lower therapeutic index in darkness than in light, this is approximately equivalent to 5.76 mg/kg over an 18 hour period.

The thiazine dyes can also be delivered using techniques known to those skilled in the art of drug delivery to target specific cell types or to enhance the activity of the dye. For example, a procedure utilizing injection of photoactive drugs for cancer treatment is described by Edelson, et al., in New England J. Med. 316, 297-303 (1987). Thiazine dyes can be specifically delivered to macrophages, a site of high HIV concentration in AIDS patients, using techniques such as liposome delivery. Liposomes are generally described by Gregoriadis, Drug Carriers in Biology and Medicine Ch. 14, 287-341 (Academic Press, NY, 1979). Methods for making light sensitive liposomes are described by Pidgeon, et al., in Photochem.Photobiol. 37, 491-494 (1983). Liposome compositions are commercially available from companies such as the Liposome Company, Inc., Princeton, NJ. Release of compounds from liposomes ingested by macrophages is described by Storm, et al., in Biochim.Biophys.Acta 965, 136-145 (1988).

Alternatively, the dye can be continuously delivered to a patient over an extended period of time using a controlled release polymeric implant. Polymeric implants are generally manufactured from polymers which degrade in vivo over a known period of time. Examples of useful polymers include polyanhydrides, polylactic acid, polyorthoester, and ethylene vinyl acetate. These devices are also commercially available. Alza Corporation, Palo Alta, CA, and Nova Pharmaceuticals, Baltimore, MD, both manufacture and distribute biodegradable controlled release polymeric devices.

Examples of useful xanthene dyes are rose bengal and eosin Y. These dyes are commercially available from a number of different sources. Rose bengal can be obtained from Sigma Chemical Co., St. Louis, Mo., while eosin Y is available from Kodak Laboratory and Research Products, Rochester, N.Y.

These compositions and methods of use thereof will be further understood by reference to the following non-limiting examples.

### Example 1: Inactivation of RNA virus R17 using methylene blue and light.

Exposure of the RNA virus R17 to 0.02 µM methylene blue plus light for 15 min (100 watt incandescent bulb at 11 cm) causes inactivation of R17, as assessed by its ability to form plaques on a bacterial lawn. The infectivity of the virus is inactivated 50% by a 45 seconds exposure to light in the presence of 0.05 µM methylene blue.

Add 20 µl (R17 phage) stock to 2.0 ml dilution buffer and dispense 0.3 ml into each of tubes A-F. Next add methylene blue to give concentrations shown in Table 1 below. Pipet 270 µl of samples to be light treated into the #1 wells of a 96 well microtiter plate, corresponding to its letter label A-H. The samples in the microtiter plate were exposed to light through water (1/4 cm) deep in a petri dish for 5 minutes. 270 µl of each sample not to be light treated was then added to wells in the plate.

After treatment, viable R17 phage were titered by making serial 10x dilutions in the microtiter plate, adding 0.1 ml of selected dilution to 0.2 ml of log phase *E. coli* strain XL-Blue cells (approximately 10⁷ cells/ml), obtained from Stratagene, La Joya, CA, and plating out 0.1 ml.

**TABLE 1**

| **Effect of methylene blue and light on the infectivity of RNA virus R17.** | | |
|---|---|---|
| MB conc. (µM) | Light 5' | pfu/ml |
| 0 | no | 7.20 x 10¹¹ |
| 0.02 | yes | 5.04 x 10¹⁰ |
| 0.04 | yes | 1.11 x 10¹⁰ |
| 0.2 | yes | 3.25 x 10⁵ |
| 2.0 | no | 4.80 x 10¹⁰ |
| 2.0 | yes | 3.00 x 10³ |
| 20.0 | no | 3.00 x 10⁹ |
| 20.0 | yes | 4.20 x 10⁴ |

The methylene blue did not inhibit growth of the bacterial lawn at the concentrations used in the R17 inactivation and titering.

### Example 2: Effect of duration of light exposure on the inactivation of R17 virus.

R17 virus samples were also tested to determine the time of exposure of light required to inactivate the virus. The procedure was similar to that used for Example 1 as outlined above except that the concentration of MB was held constant at 0.05 µM while the time of exposure was varied. The length of exposure was varied as follows:

Add 0.3 ml to well H2, treat with light 0 - 5 min. Add 0.3 ml to well G2, treat with light 5 min -7.5 min. Add 0.3 ml to well F2, treat with light 7.5 min - 9.0 min. Add 0.3 ml to well E2, treat with light 9.0 min - 9.5 min. Add 0.3 ml to well D2, treat with light 9.5 min - 9.9 min. Add 0.3 ml to well C2, treat with light 9.9 min - 10.0 min. Add 0.3 ml to well B, no light. Add 0.3 ml to well A, no light. The results are shown in Table 2.

**TABLE 2**

| **Effect of time of exposure to light on infectivity of R17 virus treated with MB.** | |
|---|---|
| Time (Min.) | PFU (Plaque Forming Units) |
| none | 2.76 x 10⁹/ml |
| 0.1' | 3.3 x 10⁹/ml |
| 0.5' | 5.0 x 10⁹/ml |
| 1.0' | 1.0 x 10⁸/ml |
| 2.5' | 9.1 x 10⁷/ml |
| 5.0' | 6.93 x 10⁷/ml |
| 10.0' | 4.35 x 10⁶/ml |

These results demonstrate that methylene blue and light inactivated R17 under these conditions at a rate of t_{1/2} = 0.8 - 1.0 minutes.

### Example 3: Anti-viral Effect of methylene blue on human immunodeficiency virus (HIV), Compared with AZT.

Methylene blue was prepared as described above. AZT was synthesized and purified by a modification of the method of Lin and Prusoff (Lin, T. -S, and W.H. Prusoff, J. Med. Chem. 21, 109-112 (1978). Acyclovir (ACV) was obtained from the Burroughs-Wellcome Co.

### HIV Antiviral Studies.

**Cells:** Human peripheral blood mononuclear cells (PBMC) from healthy HIV-1 seronegative and hepatitis B virus seronegative donors were isolated by Ficoll-Hypaque discontinuous gradient centrifugation at 1,000 x g for 30 minutes, washed twice in phosphate-buffered saline (pH 7.2; PBS), and pelleted at 300 x g for 10 minutes. Before infection, the cells were stimulated by phytohemagglutinin (PHA) at a concentration of 16.7 µg/ml, and 4 mM sodium bicarbonate buffer.

**Retrovirus:** HIV-1 (strain LAV-1) was obtained from Dr. P. Feorino (Centers for Disease Control, Atlanta, GA). The virus was propagated in human PBMC using RPMI 1640 medium, as described previously by McDougal, et al., ("Immunoassay for the detection and quantitation of infectious human retrovirus, lymphadenopathy-associated virus (LAV)," J. Immun. Meth. 76, 171 - 183, 1985) without PHA or fungizone and supplemented with 7% (v/v) interleukin-2 (Advanced Biotechnologies, Silver Spring, MD), 7 µg/ml DEAE-dextran (Pharmacia, Uppsala, Sweden), and 370 U/ml anti-human leukocyte (alpha) interferon (ICN, Lisle, IL). Virus obtained from cell-free culture supernatant was titrated and stored in aliquots at -70°C until use.

**Inhibition of Virus Replication in Human PBMC:** Uninfected PHA-stimulated human PBMC were uniformly distributed among 25 cm² flasks to give a 5 ml suspension containing about 2 x 10⁶ cells/ml. Suitable dilutions of virus were added to infect the cultures. The mean reverse transcriptase (RT) activity of the inocula was 50,000 dpm/ml corresponding to about 100 TCID₅₀, as determined by Groopman, et al., (1987), "Characterization of Serum Neutralization Response to the Human Immunodeficiency Virus (HIV)," AIDS Res. Human Retro. 3, 71-85. The drugs, at twice their final concentrations in 5 ml of RPMI 1640 medium (supplemented as described above), were added to the cultures. Uninfected and untreated PBMC at equivalent cell densities were grown in parallel as controls. The cultures were maintained in a humidified 5% CO₂-95% air incubator at 37°C for six days after infection at which point all cultures were sampled for supernatant RT activity. Previous studies had indicated that maximum RT levels were obtained at that time.

**RT Activity Assay:** A six milliliter aliquot of supernatant from each culture was clarified of cells at 300 x g for 10 minutes. Virus particles were pelleted from 5 ml samples at 40,000 rpm for 30 minutes using a Beckman 70.1 Ti rotor and suspended in 200 µl of virus disrupting buffer (50 mM Tris-HCl, pH 7.8, 800 mM NaCl, 20% glycerol, 0.5 mM phenylmethyl sulfonyl fluoride, and 0.5% Triton X-100).

The RT assay was performed in 96-well microtiter plates, as described by Spira, et al. (1987), "Micromethod for Assaying the Reverse Transcriptase of LAV-HTLV-III/Lymphadenopathy-Associated Virus," J. Clin. Microbiol. 25, 97-99. The reaction mixture, which contained 50 mM Tris-HCl, pH 7.8, 9mM MgCl₂, 5 mM dithiothreitol, 4.7 µg/ml (rA)ₙ·(dT)₁₂₋₁₈, 140 µM dATP, and 0.22 µM [³H]TTP (specific activity 78.0 Ci/mmol, equivalent to 17,300 cpm/pmol; NEN Research Products, Boston, MA), was added to each well. The sample (20 µl) was added to the reaction mixture and incubated at 37°C for 2 hours. The reaction was terminated by the addition of 100 µl 10% trichloroacetic acid (TCA) containing 0.45 mM sodium pyrophosphate. The acid-insoluble nucleic acids which precipitated were collected on glass filters using a Skatron semi-automatic harvester. The filters were washed with 5% TCA and 70% ethanol, dried, and placed in scintillation vials. Four ml of scintillation fluid (Econofluor, NEN Research Products, Boston, MA) were added and the amount of radioactivity in each sample was determined using a Packard Tri-Carb liquid scintillation analyzer (model 2,000CA). The results were expressed in dpm/ml of original clarified supernatant. The procedures for the anti-HIV-1 assays in PBMC described above have been published recently (see Schinazi, et al., Antimicrob. Agents Chemother. 32, 1784-1789, December 1988).

**HSV Preparation:** Confluent HEp-2 cells, in a roller bottle (Falcon, 850 cm²), were infected at an input multiplicity of 0.01 PFU (Plaque Forming Unit) per cell (to minimize production of defective viruses). During the two-hour absorption period, the cells were exposed to the virus inoculum diluted in absorption buffer (10 ml PBS containing 1% newborn calf serum and 0.1% glucose). The virus inoculum was then removed and replaced with Hanks' Minimum Essential Medium (MEM) containing 2% inactivated newborn calf serum, penicillin (100 U/ml) and streptomycin (100 µg/ml) (P and S), sodium bicarbonate (2 g/L) and HEPES (25 mM) (maintenance medium). The infected cells were incubated at 37°C for 3 to 4 days, until the cells could be easily shaken from the plastic surface of the culture bottles. The cells were then collected by centrifugation, suspended in a small volume of spent culture fluid (5 ml per 2 x 10⁸ cells) and sonicated 3 times for one min on ice. The disrupted cells were centrifuged (2,000 g for 15 min at 4°C and the supernatant was diluted with an equal volume of sterile skimmed milk (as stabilizer) and aliquots were frozen at - 70°C.

**HSV Plaque Reduction Assay:** Near confluent Vero (African Green Monkey) cells in 6-well plates were infected with 100 µl virus diluted in absorption buffer to give 100-200 plaques per well. The plates were then incubated at 37°C for 1 hour with intermittent rocking every 15 minutes. The inoculum was aspirated and the compounds at different concentrations (dissolved in maintenance medium) were added to replicate wells. For these assays, 0.1% pooled human gamma globulin was included in the media. The plates were placed in 5% CO₂-95% air incubator and the plaques were allowed to develop for 48 h prior to fixation (buffered 10% Formalin acetate), staining (0.5% crystal violet in 20% EtOH/H₂O) and enumeration. The degree of inhibition (per cent plaques of control) was calculated by counting the mean plaque counts for the different drug dilutions. The antiviral potency of the drugs was determined by estimating the EC₅₀, the drug concentration necessary to reduce the number of plaques by 50% relative to the virus control cultures. For routine antiviral drug screening, we used the F strain of HSV-1 and the G strain of HSV-2 (Ejercito, et al., "Characterization of Herpes Simplex Virus Strains Differing in Their Effect on Social Behavior of Infected Cells," J. Gen. Virol. 2, 357-364 (1968)). For HSV plaquing, Vero cells (rather than a human cell line such as fibroblasts) were used since these cells do not induce interferon. Acyclovir (ACV) was used as a positive control for the studies on HSV.

### Cytotoxicity Studies.

**Toxicity in Vero (African Green Monkey) Cells:** Vero cells in growth medium (2.5 ml) were added to 25 cm² flasks (Falcon) in duplicate at a concentration equivalent to one-tenth of cell confluency for each compound under test. After incubation at 37°C in 5% CO₂-95% air for 24 hr, the test compound (2x final concentration), dissolved in 2.5 ml of the growth medium was added, and two flasks were harvested immediately by decanting the medium, washing once with 3 ml of PBS, and then incubating at 37°C for 5 min with 3 ml of trypsin/EDTA (0.125%/0.02%). The cells dislodged from the flask were generally in clumps and were dispersed by repeated forceful pipetting of the suspension against the surface of the flask. To 1 ml of the well-dispersed cell suspension, 0.2 ml of trypan blue solution was added, and the number of cells were counted using a hemacytomer. Each day for the next 3 days, two of the remaining flasks were harvested in the manner just described for determination of cell number. Only data on day three are presented. This method has previously been described by Schinazi, et al., "Effect of Combination of Acyclovir, and Vidarabine or its 5'-monophosphate on Herpes Simplex Viruses in Cell Culture and in Mice," Antimicrob. Agents Chemother. 22, 499-507 (1982)).

**PBM and CEM Cells Proliferation Assay:** The drugs were evaluated for their potential toxic effects on uninfected PHA-stimulated human PBM dells and also in CEM cells. The cells were cultured with and without drug for 6 days at which time aliquots were counted for cell viability as described above.

**Enzyme Assays:** The preparation of the enzyme and the assay conditions used, were recently described by Schinazi, et al., Antimicrob. Agents Chemother. 33, 115-117 (1989). HIV-1 RT and cellular DNA polymerase alpha were isolated from infected and uninfected PHA-stimulated human PBM cells according to traditional methods (Eriksson, B., et al., Antimicrob. Agents Chemother. 31, 600-604, (1987); Furman, et al., Proc. Natl. Acad. Sci. USA 83, 8333-8337, (1986); Abrell, et al., J. Virol. 12, 431-439, (1973).

**Assays.** Reaction mixtures (100 µl) contained:

| | RT | DNA pol. alpha |
|---|---|---|
| Tris-HCl, pH 8.0 | 100 mM | 100 mM |
| KCl | 50 mM | - |
| MgCl₂ | 2 mM | 6 mM |
| DTT | 5 mM | 5 mM |
| BSA [³H]dTTP, | 400 g/ml | 400 g/ml |
| (Sp.act. 82.3 Ci/mmol) | 1 µM | 1 µM |
| dATP, dCTP, dGTP | - | 100 µM |
| Poly(rA)-oligo(dT)₁₂₋₁₈ | 3 µg/ml | - |
| Activated DNA | - | 200 µg/ml |

The reactions were started by the addition of 10 µl of purified enzyme, incubated at 37°C for the indicated periods of time and processed as described in Eriksson, et al., Antimicrob. Agents Chemother. 31, 600-604 (1987).

**Median-Effect Method:** EC₅₀ and IC₅₀ values were obtained by analysis of the data using the median-effect equation (Chou, T., et al., "Quantitative Analysis of Dose-Effect Relationships: The Combined Effects of Multiple Drugs or Enzyme Inhibitors," Adv. Enz. Regul. 22, 27-55 (1984).

### Results.

The effect of the thiazine dye methylene blue on cellular growth and proliferation was tested using PBM, Vero and CEM cell cultures. The cytotoxicity of MB, both in the presence and absence of light, was compared with that of AZT. The antiviral effect of MB was also examined using HIV-1, HSV-1 and HSV-2. The combined results of the cytotoxic and antiviral studies are shown in Table 3.

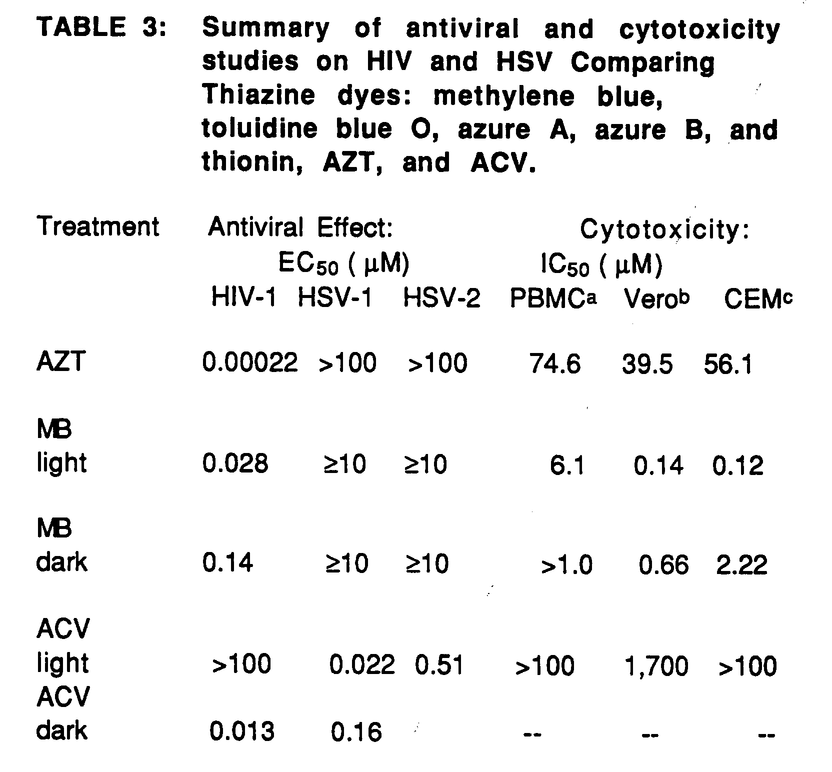

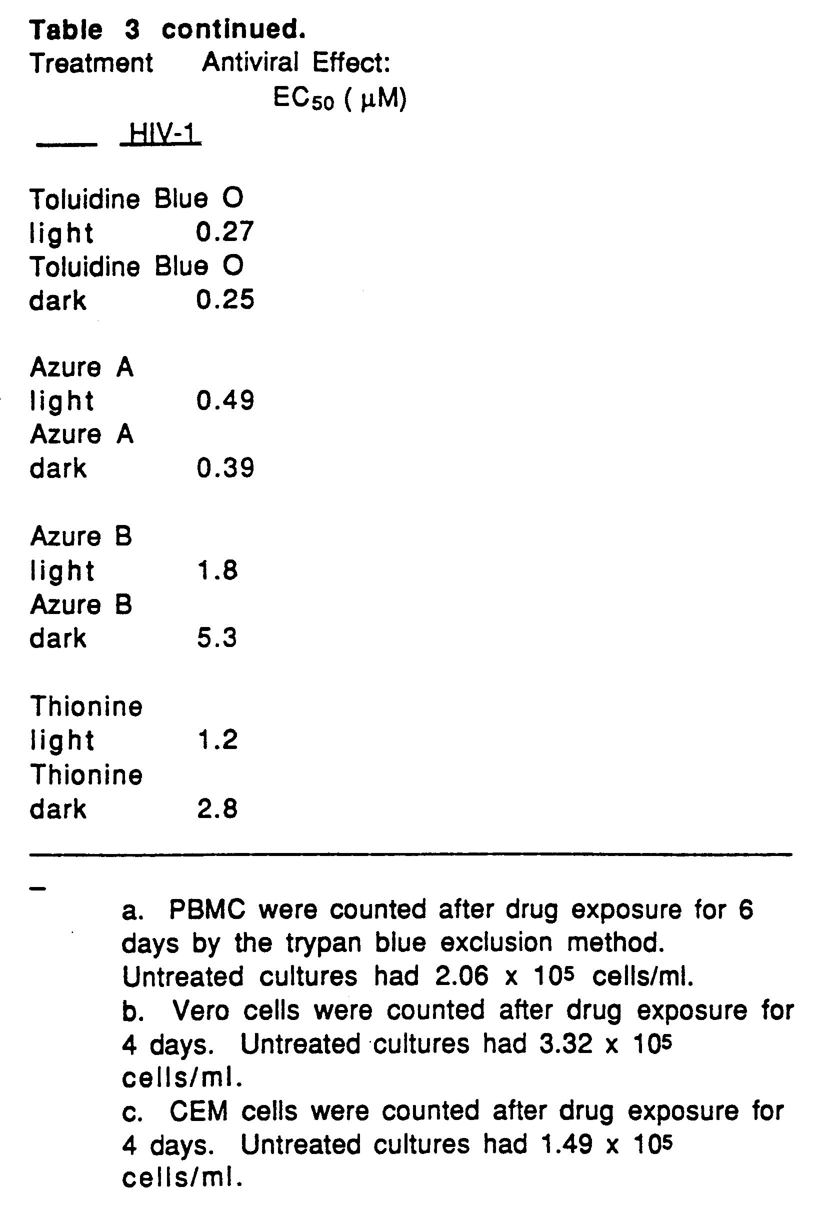

### Example 4: Measurement of P24 to quantitate inactivation of HIV by methylene blue.

The efficacy of methylene blue as an antiviral agent was further demonstrated using an independent assay method. While the previous example measured RT activity, the present example utilizes a direct quantitation of a viral protein (P24) as an indicator of antiviral effectiveness.

Peripheral blood mononuclear (PBM) cells were infected with HIV and treated with methylene blue as described above. The EC₅₀ levels were calculated from enzyme immuno-assay (EIA) measurements of viral coat protein P24. The P24-specific EIA kit was purchased from Abbott Labs. As in Example 3, the effectiveness of methylene blue was compared with AZT, the results are shown in Table 4.

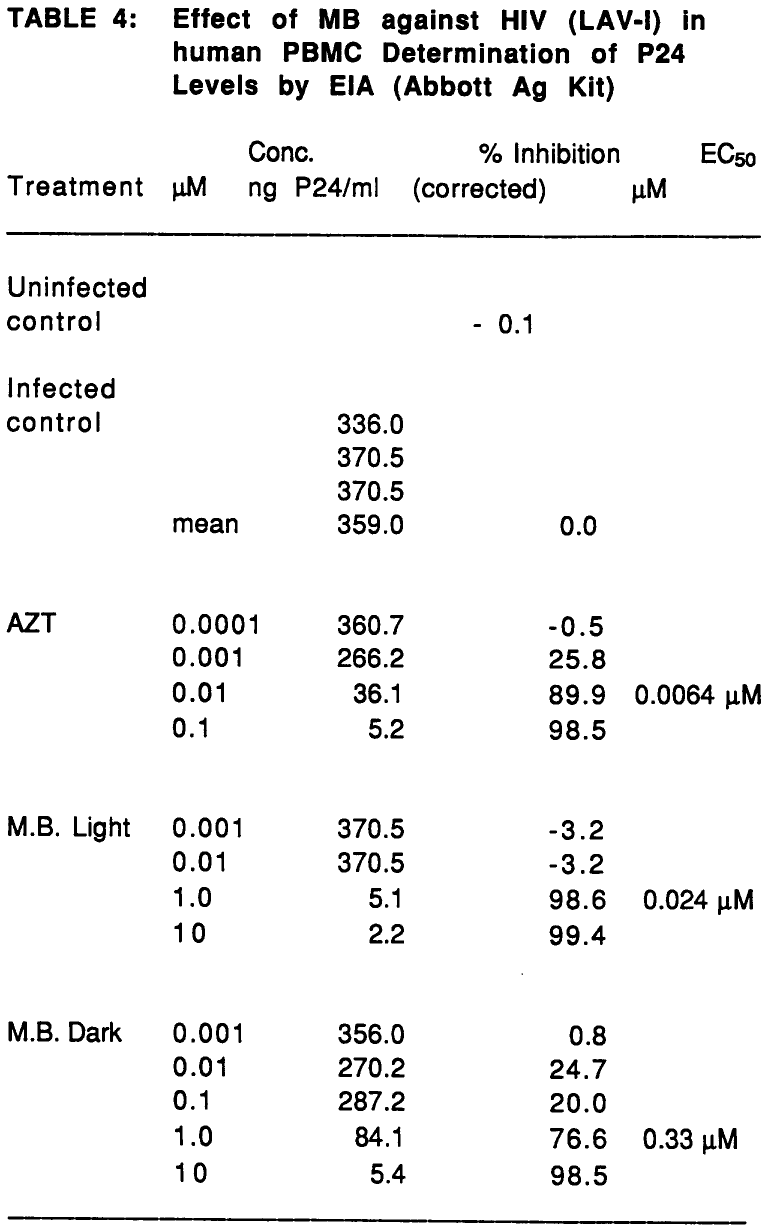

### Example 5: The effect of methylene blue on isolated DNA polymerase alpha and HIV reverse transcriptase.

The effect of methylene blue on two DNA polymerizing enzymes, HIV reverse transcriptase (RT) and calf thymus DNA polymerase alpha (CT α pol), was also studied. The results clearly show that MB is effective at inhibiting reverse transcriptase mediated DNA polymerization. In order to inhibit DNA polymerase alpha directed DNA synthesis, ten fold more MB is required, as shown in Table 5.

**TABLE 5**

| **Effect of MB on HIV-1 reverse transcriptase (RT) and calf thymus DNA polymerase in the presence and absence of light.** | | |
|---|---|---|
| Enzyme | IC₅₀ ( M) | |
| | Light | Dark |
| - | | |
| HIV-1 RT | 9.2 | 10.0 |
| CT α pol. | 125 | 105.1 |

### Example 6: Anti-viral activity of rose bengal and eosin Y.

The antiviral activity of the xanthene dyes was tested using the same reagents and assays as the methylene blue and othe thiazine dyes.

**Results:** The EC₅₀ of rose bengal in the presence of light is 1.41 µM, while in the absence of light, the EC₅₀ is 2.38 µM. The EC₅₀ of eosin Y in the presence of light is greater than 100 µM, while in the absence of light, the EC₅₀ is greater than or equal to 10 µM.

### Example 7: The toxicity of rose bengal and eosin Y in Vero (African Green Monkey) Cells.

The toxicity of the xanthene dyes was tested on Vero green monkey cells using the same reagents and assay as for the thiazine dyes.

**Results:** The effect of the compounds on the growth of uninfected Vero cells in culture is used as an indicator of the toxicity of the test compound to the normal viability of cells. The IC₅₀ is the concentration of compound which inhibits 50% of normal, uninfected cell growth. Rose bengal was determined to have an IC₅₀ of 158 µM, while eosin Y was determined to have an IC₅₀ of greater than 200 µM.

### Example 8: Toxicity of rose bengal and eosin Y in peripheral blood mononuclear cells

Rose bengal and eosin Y were evaluated for their potential toxic effect on uninfected, mitogen-stimulated peripheral blood mononuclear (PBM) cells (3.8 x 10⁵ cells/ml), as described for the thiazine dyes.

**Results:** As for the Vero cell assay, the IC₅₀ for rose bengal and eosin Y was determined in the cultured PBM cells. The IC₅₀ for rose bengal was greater than 200 µM, and for eosin Y, it was greater than 100 µM.

### Example 9: Preparation of pharmaceutical compositions.

The method described herein for the inhibition of HIV infection*s in vivo* requires dosages in the range producing a blood concentration of approximately 20 to 200 micromolar. In order to achieve this concentration, the dyes can be delivered using techniques known to those skilled in the art of drug delivery to target specific cell types or to enhance the activity of the dye.

Oral administration of the dyes is the preferred route of delivery. The concentration of active compound in the drug composition will depend on absorption, inactivation, and excretion rates of the active compound as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions. The active ingredient may be administered at once, or may be divided into a number of smaller doses to be administered at varying intervals of time.

Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents. The compounds can also be administered as a component of an elixir, suspension, syrup, wafer, chewing gum or the like. A syrup may contain, in addition to the active compounds, sucrose as a sweetening agent and certain preservatives, dyes, colorings, and flavors.

The active materials can also be mixed with other active materials which do not impair the desired action, or with materials that supplement the desired action, such as antibiotics, antifungals, antiinflammatories, or other antivirals, including other nucleoside antiviral or anticancer compounds.

While oral administration is the preferred route of delivery, there are other effective ways of delivering the dyes to the specific targets. For example, the dyes can be administered intravenously using methods known to those skilled in the art, such as the procedure utilizing injection of photoactive drugs for cancer treatment described by Edelson, et al., in New England J. Med. 316, 297-303 (1987). Ddyes can be specifically delivered to macrophages, a site of high HIV concentration in AIDS patients. Techniques such as liposome delivery can be used to attain an effective intracellular concentration of xanthene dye of approximately 0.1 to 10 micromolar. Liposomes are generally described by Gregoriadis, Drug Carriers in Biology and Medicine Ch. 14, 287-341 (Academic Press, NY, 1979). Methods for making light sensitive liposomes are described by Pidgeon, et al., in Photochem.Photobiol. 37, 491-494 (1983). Liposome compositions are commercially available from companies such as the Liposome Company, Inc., Princeton, NJ. Release of compounds from liposomes ingested by macrophages is described by Storm, et al., in Biochim.Biophys.Acta 965, 136-145 (1988).

Alternatively, the dye can be continuously delivered to a patient over an extended period of time using a controlled release polymeric implant. Polymeric implants are generally manufactured from polymers which degrad*e in vivo* over a known period of time. Examples of useful polymers include polyanhydrides, polylactic acid, polyorthoester, and ethylene vinyl acetate. These devices are also commercially available. Alza Corporation, Palo Alta, CA, and Nova Pharmaceuticals, Baltimore, MD, both manufacture and distribute biodegradable controlled release polymeric devices.

### Example 10: In Vitro Antiviral Applications

The compositions described herein for the inhibition of HIV replication can be applied in vitro to purify and remove viruses from blood products used in the laboratory and for research purposes. This application decreases the risk of laboratory workers being exposed to potentially lethal viral infections.

The dyes can also be incorporated into cleanser and disinfectant solutions at a concentration of greater than 200 micromolar.

Modifications and variations of the compositions and methods of use thereof to selectively, and in a controlled manner, inhibit specific viruses such as HIV using compositions containing xanthene dyes will be obvious to those skilled in the art from the foregoing detailed description. Such modifications and variations are intended to come within the scope of the appended claims.

## Claims

1. The use of a xanthene or thiazine dye in the manufacture of a medicament for selectively inactivating HIV, *in vivo,* or *in vitro,* without significant toxicity to cells or a patient.

2. The use according to claim 1 wherein the dye is a fluorone or hydroxyxanthane compound.

3. The use according to claim 2 wherein the dye is rose bengal or eosin Y or a combination or derivative thereof.

4. The use according to claim 1 wherein the dye is toluidine blue O, azure A, azure B, azure C, or a combination or derivative thereof.

5. The use according to claim 3 wherein the medicament provides virally-infected cells with an amount of dye yielding an intracellular concentration of between 0.1 and 10 µM.

6. The use according to claim 1 wherein the dye is methylene blue, eosin Y or rose bengal and the medicament provides an amount of dye yielding a blood concentration in a patient of between 20 and 200µM.

7. The use according to any one of the preceding claims wherein the medicament is provided in combination with non-ionising radiation.

8. The use according to any one of the preceding claims wherein the medicament comprises additionally a solution selected from a cleansing solution, antibacterial solution, antifungal solution and a biological solution.

9. The use according to claim 8 wherein the biological solution is derived from blood.

10. A method of inhibiting HIV replication in cells exposed to the virus *ex vivo,* comprising exposing the cells to a xanthene or thiazine dye, without significant toxicity to the cells.

11. A method according to claim 10 wherein the dye is methylene blue, eosin Y or rose bengal and the cells are exposed to a concentration of dye greater than 200µM.

12. A method according to claim 10 or claim 11 wherein the cells are present in blood or in a blood component.

13. A method according to any one of claims 10 to 12 wherein the cells are exposed to non-ionising radiation.

## Patentansprüche

1. Verwendung eines Xanthen- oder Thiazinfarbstoffs für die Herstellung eines Arzneimittels zur selektiven Inaktivierung von HIV *in vivo* oder *in vitro* ohne signifikante Toxizität für Zellen oder einen Patienten.

2. Verwendung nach Anspruch 1, wobei der Farbstoff eine Fluoron- oder Hydroxyxanthenverbindung ist.

3. Verwendung nach Anspruch 2, wobei der Farbstoff Bengalrosa oder Eosin Y oder eine Kombination oder ein Derivat davon ist.

4. Verwendung nach Anspruch 1, wobei der Farbstoff Toluidinblau O, Azur A, Azur B, Azur C oder eine Kombination oder ein Derivat davon ist.

5. Verwendung nach Anspruch 3, wobei das Arzneimittel virusinfizierte Zellen mit einer Farbstoffmenge bereitstellt, die eine intrazelluläre Konzentration zwischen 0,1 und 10 µM liefert.

6. Verwendung nach Anspruch 1, wobei der Farbstoff Methylenblau, Eosin Y oder Bengalrosa ist und das Arzneimittel eine Farbstoffmenge bereitstellt, die eine Blutkonzentration zwischen 20 und 200µM in einem Patienten liefert.

7. Verwendung nach einem der vorstehenden Ansprüche, wobei das Arzneimittel in Kombination mit einer nicht-ionisierenden Strahlung bereitgestellt wird.

8. Verwendung nach einem der vorstehenden Ansprüche, wobei das Medikament zusätzlich eine Lösung, ausgewählt aus einer Reinigungslösung, antibakteriellen Lösung, antifungalen Lösung und einer biologischen Lösung, umfaßt.

9. Verwendung nach Anspruch 8, wobei die biologische Lösung aus Blut stammt.

10. Verfahren zur Inhibierung der HIV-Replikation in dem Virus ausgesetzten Zellen *ex vivo,* umfassend das Aussetzen der Zellen einem Xanthen- oder Thiazinfarbstoff ohne signifikante Toxizität für die Zellen.

11. Verfahren nach Anspruch 10, wobei die Farbe Methylenblau, Eosin Y oder Bengalrosa ist und die Zellen einer Farbstoffkonzentration von größer als 200 µM ausgesetzt werden.

12. Verfahren nach Anspruch 10 oder 11, wobei die Zellen im Blut oder in einem Blutbestandteil vorhanden sind.

13. Verfahren nach einem der Ansprüche 10 bis 12, wobei die Zellen einer nicht-ionisierenden Strahlung ausgesetzt werden.

## Revendications

1. Utilisation d'un colorant à base de xanthène ou de thiazine dans la préparation d'un médicament pour inactiver sélectivement le HIV, in vivo ou in vitro, sans exposer à une toxicité notable des cellules ou un patient.

2. Utilisation selon la revendication 1, dans laquelle le colorant est un composé à base de fluorone ou d'hydroxyxanthane.

3. Utilisation selon la revendication 2, dans laquelle le colorant est le rose bengale ou l'éosine Y ou une de leurs combinaisons ou un de leurs dérivés.

4. Utilisation selon la revendication 1, dans laquelle le colorant est le bleu de toluidine O, le bleu azur A, le bleu azur B, le bleu azur C ou une de leurs combinaisons ou un de leurs dérivés.

5. Utilisation selon la revendication 3, dans laquelle le médicament délivre à des cellules infectées d'un virus une quantité de colorant donnant une concentration intracellulaire entre 0,1 et 10 µM.

6. Utilisation selon la revendication 1, dans laquelle le colorant est le bleu de méthylène, l'éosine Y ou le rose bengale et le médicament délivre une quantité de colorant donnant une concentration dans le sang d'un patient entre 20 et 200 µM.

7. Utilisation selon l'une quelconque des revendications précédentes, dans laquelle le médicament est délivré en combinaison avec un rayonnement non ionisant.

8. Utilisation selon l'une quelconque des revendications précédentes, dans laquelle le médicament comprend en outre une solution choisie parmi une solution de purification, une solution antibactérienne, une solution antifongique et une solution biologique.

9. Utilisation selon la revendication 8, dans laquelle la solution biologique est dérivée du sang.

10. Procédé d'inhibition de la réplication du HIV dans des cellules exposées au virus ex vivo, consistant à exposer les cellules à un colorant à base de xanthène ou de thiazine sans exposer les cellules à une toxicité notable.

11. Procédé selon la revendication 10, dans lequel le colorant est le bleu de méthylène, l'éosine Y ou le rose bengale et les cellules sont exposées à une concentration de colorant supérieure à 200 µM.

12. Procédé selon la revendication 10 ou 11, dans lequel les cellules sont présentes dans le sang ou dans un composant du sang.

13. Procédé selon l'une quelconque des revendications 10 à 12, dans lequel les cellules sont exposées à un rayonnement non ionisant.
